(19) Europäisches Patentamt
European Patent Office
Office européen des brevets

(11) Veröffentlichungsnummer: **0 003 974**
**B1**

(12) # EUROPÄISCHE PATENTSCHRIFT

(45) Veröffentlichungstag der Patentschrift: **15.04.81**

(21) Anmeldenummer: **79100421.1**

(22) Anmeldetag: **13.02.79**

(51) Int. Cl.³: **C 07 D 277/42, C 07 D 417/06, A 01 N 43/78**

(54) **Trifluormethylimino-thiazolidin-Derivate, Verfahren zu ihrer Herstellung und ihre Verwendung als Fungizide.**

(30) Priorität: **25.02.78 DE 2808227**

(43) Veröffentlichungstag der Anmeldung:
**19.09.79 Patentblatt 79/19**

(45) Bekanntmachung des Hinweises auf die Patenterteilung:
**15.04.81 Patentblatt 81/15**

(84) Benannte Vertragsstaaten:
**BE CH DE FR GB IT NL SE**

(56) Entgegenhaltungen:
**DE - A - 2 062 346**
**DE - A - 2 062 348**
**DE - A - 2 210 882**

(73) Patentinhaber: **BAYER AG**
**Zentralbereich Patente, Marken und Lizenzen**
**D-5090 Leverkusen 1, Bayerwerk (DE)**

(72) Erfinder: **Grohe, Klaus, Dr.**
**Am Wasserturm 10**
**D-5068 Odenthal (DE)**
Erfinder: **Scholl, Hans-Joachim, Dr.**
**Rudolf-Sohm-Strasse 28**
**D-5000 Köln 60 (DE)**
Erfinder: **Paul, Volker, Dr.**
**Ahornstrasse 5**
**D-5650 Solingen 1 (DE)**
Erfinder: **Brandes, Wilhelm, Dr.**
**Eichendorffstrasse 3**
**D-5653 Leichlingen 1 (DE)**
Erfinder: **Frohberger, Paul-Ernst, Dr.**
**Willi-Baumeister-Strasse 5**
**D-5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 003 974 B1

# Trifluormethylimino-thiazolidin-Derivate, Verfahren zu ihrer Herstellung und ihre Verwendung als Fungizide

Die vorliegende Erfindung betrifft neue Trifluormethylimino-thiazolidin-Derivate, ein Verfahren zu ihrer Herstellung sowie ihre Verwendung als Fungizide.

Wie bekannt ist, verwendet man zur Bekämpfung pflanzenschädigender Pilze in weitem Umfang das Zink-äthylen-bis-dithiocarbamat (vgl. Phytopathology *33,*, 1113 (1943)), die Wirkung dieses Stardardpräparates ist jedoch nicht immer voll befriedigend. Auch die ebenfalls bekannten 4,5-Bis-trifluormethylimino-thiazolidine besitzen eine gute fungizide Wirksamkeit (vgl. hierzu DT—OS 2 062 348 [Le A 13 420]), aber auch hier zeigen sich bei niedrigen Aufwandmengen nicht immer voll zufriedenstellende Wirkungen.

Es wurden nun als neue Stoffe die Trifluormethylimino-thiazolidin-Derivate der allgemeinen Formel

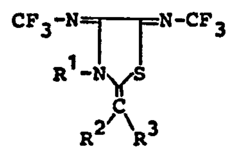

(I)

in welcher

$R^1$ für Alkyl, Alkenyl und Alkinyl steht, wobei diese Gruppen gegebenenfalls substituiert sein können durch Cyano-, Alkoxy- und Alkylmercapto-Gruppen mit jeweils bis zu 3 Kohlenstoffatomen, ferner für gegebenenfalls durch Alkyl mit bis zu 3 Kohlenlstoffatomen substituiertes Cycloalkyl steht, ferner für Aryl und Aralkyl steht, wobei die beiden letztgenannten Gruppen am Aryl-Rest substituiert sein können durch Halogen-, Cyano-, Nitro-, Alkyl-, Alkoxy- und Alkylmercapto-Gruppen mit jeweils bis zu 3 Kohlenstoffatomen, und endlich für eine Amino-Gruppe —$NR^4R^5$ steht, wobei

$R^4$ und $R^5$ für Alkyl stehen, wobei die Reste $R^4$ und $R^5$ auch gemeinsam mit dem verbindenden Stickstoffatom und gegebenenfalls noch weiteren Heteroatom, wie Sauerstoff, Schwefel oder Stickstoff, einen 4- bis 12-gliedrigen heterocyclischen Ring bilden können,

$R^2$ für Wasserstoff, eine Nitril-, Alkoxycarbonyl- oder Acyl-Gruppe steht, und

$R^3$ für eine Nitril- oder Alkoxycarbonyl-Gruppe steht, wobei letztere im Alkylteil substituiert sein kann durch Alkoxy- oder Alkylmercapto-Gruppen mit jeweils bis zu 3 Kohlenstoffatomen, ferner für eine Acyl-, Alkylsulfonyl- oder Arylsulfonyl-Gruppe steht, wobei letztere im Aryl-Teil substituiert sein kann durch Alkyl mit bis zu 3 Kohlenstoffatomen, Nitro-Gruppen und/oder Halogen, ferner für eine Amid- oder Thioamid-gruppe mit den Resten CO—$NR^6R^7$ bzw. CS—$NR^6R^7$ steht, wobei

$R^6$ für Wasserstoff oder Alkyl steht, und

$R^7$ für Alkyl, Cycloalkyl, Benzyl oder Phenyl steht, wobei letzteres substituiert sein kann durch Halogen, Nitro-, Alkyl-, Alkoxy- und Alkylmercapto-Gruppen mit jeweils bis zu 4 Kohlenstoffatomen im Alkylteil, und weiterhin können

$R^2$ und $R^3$ noch gemeinsam mit dem verbindenden Kohlenstoffatom für ein $\alpha$-Cycloalkanon-Ringsystem mit 5 bis 7 Kohlenstoffatomen stehen, das gegebenenfalls durch Alkyl mit bis zu 3 Kohlenstoffatomen substituiert sein kann,

gefunden. Sie weisen starke fungizide Eigenschaften auf.

Weiterhin wurde gefunden, daß man die Trifluormethylimino-thiazolidin-Derivate der Formel I erhält, wenn man Thioamide der Formel (dargestellt in der reaktionsfähigen Enthiolform)

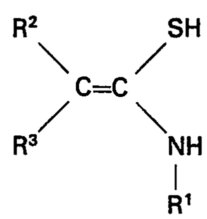

(II)

in welcher

$R^1$, $R^2$ und $R^3$ die oben angegebene Bedeutung besitzen,

mit Bis-trifluormethylimino-oxalsäuredifluorid der Formel

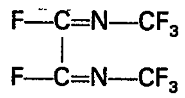 (III)

in Gegenwart eines Fluorwasserstoff-Akzeptors umsetzt.

Überraschenderweise besitzen die erfindungsgemaßen Trifluormethylimino-thiazolidin-Derivate eine höhere fungizide Wirksamkeit als die aus dem Stande der Technik bekannten Stoffe. Die erfindungsgemäßen Verbindungen stellen somit eine Bereicherung der Technik dar.

Verwendet man (N-Benzyl)-malonsäurethioamidmethylester und Bis-trifluormethylimino-oxalsäuredifluorid als Ausgangsstoffe, so kann der Reaktivablauf durch das folgende Formelschema widergegeben werden:

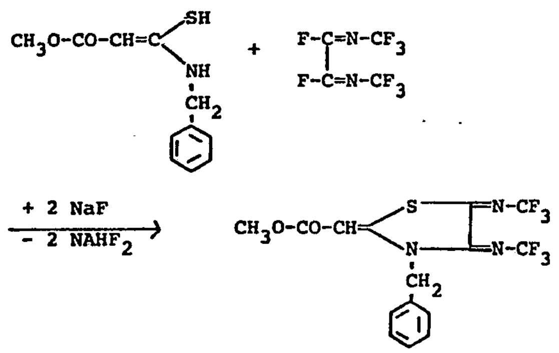


Die als Ausgangsstoffe zu verwendenen Thioamide sind durch die Formel II allgemein definiert. In dieser Formel steht $R^1$ vorzugsweise für geradkettiges oder verzweigtes Alkyl, Alkenyl und Alkinyl mit jeweils bis 6, insbesondere bis zu 4 Kohlenstoffatomen, die genannten Reste können substituiert sein durch Cyano-, Alkoxy- und Alkylmercapto-Gruppen mit jeweils bis zu 3 Kohlenlstoffatomen; weiterhin steht $R^1$ vorzugsweise für gegebenenfalls substituiertes Cycloalkyl mit 5 bis 6 Kohlenstoffatomen, als Substituenten kommen Alkyl-Gruppen mit 1 bis 3 Kohlenstoffatomen in Frage; sodann steht $R^1$ vorzugsweise für Aryl mit bis zu 10 Kohlenstoffatomen und für Aralkyl mit bis zu 10 Kohlenstoffatomen im Aryl- und 1 oder 2 Kohlenstoffatomen im Alkyl-Teil, als Substituenten der genannten Aryl-Reste kommen Halogen, die Cyano-, Nitro-, Alkyl-, Alkoxy- und Alkylmercapto-Gruppen, letztere mit jeweils bis zu 3 Kohlenstoffatomen in Frage, und endlich steht $R^1$ noch vorzugsweise für die Amino-Gruppe —$NR^4R^5$. Hierbei können $R^4$ und $R^5$ gleich oder verschieden sein und stehen für geradkettiges oder verzweigtes Alkyl mit 1 bis 12, vorzugsweise 1 bis 4 Kohlenstoffatomen, hierbei können die Alkyl-Gruppen mit dem verbindenden Stickstoffatom und gegebenenfalls weiteren Heteroatom, wie Sauerstoff, Schwefel oder Stickstoff, einen Heterocyclus mit vorzugsweise 5 bis 7 Ringatomen bilden. $R^2$ steht vorzugsweise für Wasserstoff, eine Cyano- oder Alkoxycarbonyl-Gruppe mit bis zu 4 Kohlenstoffatomen im Alkylteil, sowie für eine Acyl-Gruppe mit vorzugsweise bis zu insgesamt 10, insbesondere bis zu 4 Kohlenstoffatomen. $R^3$ steht vorzugsweise für die Cyano- und für die Alkoxycarbonyl-Gruppe mit vorzugsweise bis zu 4 Kohlenstoffatomen im Alkylteil, letztere kann gegebenenfalls substituiert sein durch eine Alkoxy- oder Alkylmercapto-Gruppe mit bis zu 3 Kohlenstoffatomen, $R^3$ steht weiterhin für eine Acyl-Gruppe mit insgesamt bis zu 10 Kohlenstoffatomen, vorzugsweise bis zu 6 Kohlenstoffatomen, ferner für eine Alkylsulfonyl- mit vorzugsweise bis zu 6 und eine Arylsulfonyl-Gruppe mit vorzugsweise bis zu 10 Kohlenstoffatomen im Aryl-Teil, wobei letztere durch Alkyl mit bis zu 3 Kohlenstoffatomen, Nitrogruppen und/oder Halogen substituiert sein kann, ferner vorzugsweise für eine Amid- oder Thioamid-Gruppe mit den Resten CO—$NR^6R^7$ bwz. CS—$NR^6R^7$, wobei $R^6$ vorzugsweise für Wasserstoff oder Alkyl mit bis zu 4 Kohlenstoffatomen steht, und $R^7$ vorzugsweise für Alkyl mit bis zu 6 und Cycloalkyl mit vorzugsweise 5 bis 6 Kohlenlstoffatomen steht und endlich für Phenyl steht, welches substistuiert sein kann durch Halogen, Nitro-, Alkyl-, Alkoxy- und Alkylmercapto-Gruppen mit jeweils bis zu 4 Kohlenstoffatomen im Alkylteil. Weiterhin können noch $R^2$ und $R^3$ gemeinsam mit dem verbindenden Kohlenstoffatom vorzugsweise für ein gegebenenfalls durch Alkyl mit bis zu 3 Kohlenstoffatomen substituiertes $\alpha$-Cycloalkanon-Ringsystem mit 5 bis 6 Kohlenstoffatomen stehen, so z.B. für eine $\alpha$-Cyclopentanon- und $\alpha$-Cyclohexanon-Ringsystem.

Die Thioamide der Formel II bzw. Thiohydrazide (in den Fällen, in denen $R^1$ für eine gegebenenfalls substituierte Amino-Gruppe steht) sind bekannte Verbindungen oder können nach allgemein bekannten Verfahren hergestellt werden. So erhält man z.B. Malonsäure-dithioamide durch Umsetzung von Malonsäureamiden mit Phosphorpentasulfid (Ber. dtsch. chem. Ges. *39*, B 298 (1906)) oder durch Entacylierung von Acetylaceton mit pro Mol Acetylaceton 2 Molen eines Senföls (J. prakt. Chem. *30*, 63 (1965) referiert in Chem. Abstracts 64, 3411 $\alpha$ (1966); Liebigs Ann. Chem. *695*, 49 (1966)). Acetessigsäurethioamide werden durch Umsetzung von Acetylaceton mit einem Mol Senföl erhalten (J.Am.Chem.Soc. *42*, 1055 (1920), Z. Chem. *16*, 452 (1976)). Nach dem gleichen Schema reagieren

auch Acetoacetamide mit aromatischen Isothiocyanaten zu (N-Aryl)-malonsäure-thioamiden (J. prakt. Chem. *34*, 251 (1966)). Entsprechend können auch aus Acetessigestern und aromatischen Senfölen (N-Aryl)-maonsäure-thioamidester hergestellt werden (Z. Chem. *5*, 104 (1965) referiert in Chem. Abstracts 63, 5546 $\alpha$ (1965), Liebigs Ann. Chem. *695*, 49 (1966). (N-Alkyl)-malonsäure-thioamidester und -thiohydrazidester können auf einfache Weise durch Umsetzung der asymmetrischen Dithiomalonester (Suomen Kemistilehti *B 17*, 28 (1944) mit primären aliphatischen Aminen bzw. 1,1-disubstituierten Hydrazinen in guter Ausbeute erhalten werden (vgl. Zh. Org. Khim. *11*, 1192 (1975). Benzoylessigsäurethioamide bzw. Cycloalkanon-(2)-thiocarbonsäureanilide können durch Acylierung von $\alpha$-Morpholinostyrol bzw. 1-Morpholino-cycloalkenen-(1) mit Senfölen und nachfolgende saure Hydrolyse synthetisiert werden (Chem. Ber. *95*, 926 (1962), referiert in Chem. Abstracts *57*, 4654 (1962). Schließlich werden Cyanessigsäure-thioanilide durch Acylierung von Cyanessigestern mit Arylsenfölen und anschließend Verseifung und Decarboxylierung der Addukte erhalten (Zh. Obsh. Khim. *32*, 2248 (1962), referiert in Chem. Abstracts *58*, 7863 f (1963); Khim. Get. Soed. *1*, 698 (1965), referiert in Chem. Abstracts 64, 9702 c (1966); Khim. Get. Soed. *3*, 713 (1967), referiert in Chem. Abstracts *68*, 49503 j (1968)).

Zur Durchführung des erfindungsgemäßen Verfahrens werden vorteilhaft solche sekundäre Thioamide verwendet, die in $\alpha$-Stellung ein bewegliches Wasserstoffatom besitzen. Die Thioamide reagieren formal in der Enthiolform, wie sie in Formel II dargestellt wurde.

Das weiterhin als Ausgangsprodukt verwendete Bis-trifluormethylimino-oxalsäuredifluorid der Formel III, auch als Perfluor-2,5-diazahexa-2,4-dien bezeichnet, ist bekannt (vgl. J. Am. Chem. Soc. *89*, 5007 (1967) oder US—PS 3660 511).

Als Verdünnungsmittel können übliche organische Lösungsund Verdünnungsmittel verwendet werden. Hierzu gehören Kohlenwasserstoffe, wie z.B. Benzin, Toluol und Cyclohexan, ferner Nitrile, wie z.B. Acetonitril und Propionitril, sodann chlorierte Kohlenwasserstoffe, wie Methylenchlorid, Chloroform und Chlorbenzol, oder Ketone, wie z.B. Aceton. Vorzugsweise wird in Aceton oder Acetonitril gearbeitet.

Als Säurebinder können übliche Säurebindungsmittel verwendet werden. Als solche können Alkalicarbonate, Alkalibicarbonate, sowie tertiäre Amine, wie Triäthylamin, Dimethylanilin oder Pyridin verwendet werden. Besonders bevorzugt werden zur Bindung des freiwerdenden Fluorwasserstoffs Alkalifluoride, wie z.B. Natriumfluorid, eingesetzt.

Die Reaktionstemperaturen können in einem größeren Bereich variiert werden. Im allgemeinen arbeitet man zwischen —50 und +120°C, vorzugsweise zwischen —30 und +90°C.

Zur Durchführung des erfindungsgemäßen Verfahrens setzt man auf 1 Mol Thioamid gemäß Formel II 1 Mol Bis-trifluormethylimino-oxalsäure-difluorid der Formel III ein. Alkalifluorid als Fluorwasserstoffakzeptor wird in etwa 2- bis 4-molarer Menge eingesetzt. Unter- oder Überschreitungen der angegebenen Mengenverhältnisse um bis zu etwa 20% können ohne wesentliche Erniedrigung der Ausbeute vorgenommen werden. Zweckmäßigerweise läßt man das Bis-trifluormethylimino-oxalsäuredifluorid zu einer Suspension bestehend aus Thioamid, organischem Lösungsmittel und Fluorwasserstoffakzeptor tropfen, wobei vorteilhafterweise zunächst mit einem Kältebad gekühlt wird. Nach beendigter Reaktion (z.B. nach einem Zeitraum von 30 Minuten bis 24 Stunden bei Raumtemperatur) wird von gebildetem Hydrogenfluorid abfiltriert, das Filtrat eingeengt und der kristalline Rückstand durch Umkristallisation gereinigt. Man kann aber auch nach beendigter Reaktion das erhaltene Gemisch oder die filtrierte Lösung in Eiswasser gießen, den anfallenden Rückstand absaugen und gegebenenfalls umkristallisieren.

Eine Variante des Verfahrens zur Herstellung der erfindungsgemäßen Verbindungen besteht darin, daß man geeignete Thioamide mit dem bekannten N,N'-Bis-(trifluormethyl)-tetrafluoräthylen-1,2-diamin in Gegenwart eines Fluorwasserstoffakzeptors im Temperaturbereich zwischen —50 und +20°C umsetzt.

In diesem Fall werden vorteilhaft pro Mol Thioamid 1 Mol N,N'-Bis-(trifluormethyl)-tetrafluoräthylen-1,2-diamin und 4—5 Mol Fluorwasserstoffakzeptor eingesetzt (diese Verfahrensvariante wird analog der in der DT—OS 2 210 882 [Le A 14 194] offenbarten Methode durchgeführt).

Die erfindungsgemäßen Wirkstoffe weisen eine starke fungitoxische Wirkung auf. Sie schädigen Kulturpflanzen in den zur Bekämpfung von Pilzen notwendigen Konzentrationen nicht. Aus diesen Gründen sind sie für den Gebrauch als Pflanzenschutzmittel zur Bekämpfung von Pilzen geeignet. Fungitoxische Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die erfindungsgemäßen Wirkstoffe haben eine breites Wirkungsspektrum und können angewandt werden gegen parasitäre Pilze, die oberirdische Pflanzenteile befallen oder die Pflanzen vom Boden her angreifen sowie gegen samenübertragbare Krankheitserreger.

Die Verbindungen besitzen eine gute Wirksamkeit gegen Fusicladium dendriticum, den Erreger des Apfelschorfs, gegen Phytophthora infestans, den Erreger der Krautund Knollenfäule der Kartoffeln und gegen Pyricularia oryzae, den Erreger der Blattfleckenkrankheit des Reises.

Die erfindungsgemäßen Verbindungen wirken jedoch auch gegen andere Pilze, die Reis- oder andere Kultur-Pflanzen befallen, wie z.B. gegen Mycosphaerella musicola, Verticillium alboatrum, Phialophora cinerescens und gegen das Bakterium Xanthomones oryzae.

Die Wirkstoffe können in die üblichen Formulierungen übergeführt werden, wie Lösungen,

Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenlwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyllähylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage; z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als fester Trägerstoffe für Granulate kommen in Frage; z.B. gebrochne und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage; z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die Wirkstoffe können als solche, in Form ihrer Formulierung oder in den daraus bereiteten Anwendungsforme, wie gebrauchsfertige Lösungen, emulgierbare Konzentrate, Emulsionen, Suspensionen, Spritzpulver, Pasten, lösliche Pulver, Stäubemittel und Granulate angewendet werden. Die Anwendung geschieht in üblicher Weise, z.B. durch Verspritzen, Versprühen, Vernebeln, Verstäuben, Verstreuen, Verräucheren, Vergasen, Gießen, Beizen oder Inkrustieren.

Die Wirkstoffkonzentrationen in den anwendungsfertigen Zubereitungen können in größberen Bereichen variiert werden. Im allgemeinen liegen sie zwischen 0,0001 und 10%, vorzugsweise zwischen 0,01 und 1%.

Die erfindungsgemäßen Verbindungen besitzen auch eine akarizide Wirkung.

Beispiel A
Agarplattentest

Verwendeter Nährboden:

20 Gewichtsteile Agar-Agar

200 Gewichtsteile Kartoffeldekokt

5 Gewichtsteile Malz

15 Gewichtsteile Dextrose

5 Gewichtsteile Pepton

2 Gewichtsteile Dinatriumhydrogenphosphat

0,3 Gewichtsteile Caciumnitrat

Verhältnis von Lösungsmittelgemisch zum Nährboden:

2 Gewichtsteile Lösungsmittelgemisch

100 Gewichtsteile Agarnährboden

Zusammensetzung Lösungsmittelgemisch

0,19 Gewichtsteile Dimethylformamid oder Aceton

0,01 Gewichtsteile Emulgator Alkylarylpolyglykoläther

1,80 Gewichtsteile Wasser

———

2 Gewichtsteile Lösungsmittelgemisch

Man vermischt die für die gewünschte Wirkstoffkonzentration im Nährboden nötige Wirkstoffmenge mit der angegebenen Menge des Lösungsmittelgemisches. Das Konzentrat wird im genannten Mengenverhältnis mit dem flüssigen, auf 42°C abgekühlten Nährboden gründlich vermischt und in Petrischalen mit einem Durchmesser von 9 cm gegossen. Ferner werden Kontrollplatten ohne Präparatbeimischung aufgestellt.

Ist der Nährboden erkaltet und fest, werden die Platten mit den unten angegebenen Pilzarten und einem Bakterium beimpft und bei etwa 21°C inkubiert.

Die Auswertung erfolgt je nach der Wachstumsgeschwindigkeit der Organismen nach 4—10 Tagen. Bei der Auswertung wird das radiale Wachstum auf den behandelten Nährböden mit dem Wachstum auf dem Kontrollnährboden verglichen. Die Bonitierung des Wachstums geschieht mit folgenden Kennzahlen:

1 kein Wachstum

bis 3 sehr starke Hemmung des Wachstums

bis 5 mitttelstarke Hemmung des Wachstums

bis 7 schwache Hemmung des Wachstums

9 Wachstum gleich der unbehandelten Kontrolle.

Die Versuchsauswertung ergab, daß z.B. die folgenden Verbindungen eine im Vergleich zu den beim Stande der Technik genannten Verbindungen überlegene Wirkung aufzeigen:

Verbindungen gemäß Herstellungsbeispielen 2, 15, 17, 19, 20, 33.

Geprüft wurde die Wirking gegen die Pilzarten Colletotrichum, Coffeanum, Cochliobolus miyabeanus, Verticillium albatrum, Pyricularia oryzae, Phialophora cinerescens, Mycosphaerella unsicola und Pellicularia sasakii und gegen das Bakterium Xanthomonas oryzae.

Beispiel B

Pyricularia- und Pellicularia-Test (Reis)

| | | |
|---|---|---|
| Lösungsmittel: | 11,75 | Gewichtsteile Aceton |
| Dispergiermittel: | 0,75 | Gewichtsteile Alkylarylpolyglykoläther |
| Wasser | 987,50 | Gewichtsteile |

Man vermischt die für die gewünschte Wirkstoffkonzentration in der Spritzflüssigkeit nötige Wirkstoffmenge mit der angegebenen Menge des Lösungsmittels und des Dispergiermittels und verdünnt das Konzentrat mit der angegebenen Menge Wasser.

Mit der Spritzflüssigkeit bespritzt man etwa 2 bis 4 Wochen alte Reispflanzen bis zu Tropfnässe. Die Pflanzen verbleiben bis zum Abtrocknen in einem Gewächshaus bei Temperaturen von 22 bis 24°C und einer relativen Luftfeuchtigkeit von etwa 70%. Danach wird der eine Teil der Pflanzen mit einer wäßrigen Suspension von 100000 bis 200000 Sporen/ml Pyricularia oryzae inokuliert und in einem Raum bei 24 bis 26°C und 100% relativer Luftfeuchtigkeit aufgestellt. Der andere Teil der Pflanzen

wird mit einer auf Malzagar gezogenen Kultur von Pellicularia sasakii infiziert und bei 28 bis 30°C sowie 100% relativer Luftfeuchtigkeit aufgestellt.

5 bis 8 Tage der Inokulation wird der Befall bei allen zur Zeit der Inokulation mit Pyricularia oryzae vorhandenen Blättern in Prozent der unbehandelten, aber ebenfalls inokulierten Kontrollpflanzen bestimmt. Bei den mit Pellicularia sasakii infizierten Pflanzen wird der Befall nach der gleichen Zeit an den Blattscheiben ebenfalls im Verhältnis zur unbehandelten, aber infizierten Kontrolle bestimmt. Die auswertung erfolgt in Wertzahlen von 1—9. 1 bedeutet 100%ige Wirkung, 3=gute Wirkung, 5=mäßige Wirkung und 9=keine Wirkung.

Die Auswertung der Versuche ergab, daß z.B die folgenden Verbindungen eine im Vergleich zu den beim Stande der Technik genannten Verbindungen überlegene Wirkung aufzeigen:

Verbindungen gemäß Herstellungsbeispielen 2, 3, 1, 15, 17, 19, 20, 33, 35.

## Beispiel C

### Phytophthora-Test (Tomaten)/Protektiv

| | | |
|---|---|---|
| Lösungsmittel: | 4,7 | Gewichtsteile Aceton |
| Emulgator: | 0,3 | Gewichtsteile Alkylaryl-polyglykoläther |
| Wasser: | 95 | Gewichtsteile |

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Tomatenpflanzen mit 2 bis 4 Laubblättern bis zur Tropfnässe. Die Pflanzen verbleiben 24 Stunden bei 20°C und einer relativen Luftfeuchtigkeit von 70% im Gewächshaus. Anschließend werden die Tomatenpflanzen mit einer wäßrigen Sporensuspension von Phytophthora infestans inokuliert. Die Pflanzen werden in eine Feuchtkammer mit einer 100%igen Luftfeuchtigkeit und einer Temperatur von 18 bis 20°C gebracht.

Nach 5 Tagen wir der Befall der Tomatenpflanzen bestimmt. Die erhaltenen Boniturwerte werden auf Prozent Befall umgerechnet. 0% bedeutet keinen Befall, 100% bedeutet, daß die Plfanzen vollständig befallen sind.

Die Versuchsauswertung ergab, daß z.B. die folgenden Verbindungen eine im Vergleich zu den beim Stande der Technik genannten Verbindungen überlegene Wirkung aufzeigen:

Verbindungen gemäß Herstellungsbeispiele 1, 9, 34, 24.

## Beispiel D

### Fusicladium-Test (Apfel)/Protektiv

| | | |
|---|---|---|
| Lösungsmittel: | 4,7 | Gewichtsteile Acetan |
| Emulgator: | 0,3 | Gewichtsteile Alkyl-aryl-polyglykoläther |
| Wasser: | 95 | Gewichtsteile |

Man vermischt die für die gewünschte Wirkstoffkonzentration in der Spritzflüssigkeit nötige Wirkstoffmenge mit der angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 Stunden bei 20°C und einer relativen Luftfeuchtigkeit von 70% im Gewächshaus. Anschließend werden sie mit einer wäßrigen Konidiensuspension des Apfelschorferregers (Fusicladium dendriticum) inokuliert und 18 Stunden lang in einer Feuchtkammer bei 18 bis 20°C und 100% relativer Luftfeuchtigkeit inkubiert.

Die Pflanzen kommen dann erneut für 14 Tage ins Gewächshaus.

15 Tage nach der Inokulation wird der Befall der Sämlinge bestimmt. Die erhaltenen Boniturwerte werden in Prozent Befall umgerechnet. 0% bedeutet keinen Befall, 100% bedeutet, daß die Pflanzen vollständig befallen sind.

Die Versuchsauswertung ergab, daß z.B. die folgenden Verbindungen eine im Vergleich zum Stande der Technik überlegene Wirkung aufzeigen:

Verbindungen gemäß Herstellungsbeispielen 1, 9, 10, 15, 33, 34, 24.

## Beispiel E

Sproßbehandlungs-Test/Getreiderost/protektiv (blattzerstörende Mykose)

Zur Herstellung einer zweckmäßigen Wirkstoffzubereitung nimmt man 0,25 Gewichtsteile Wirkstoff in 25 Gew.-Teilen Dimethylformamid und 0,06 Gewichtsteilen Emulgator Alkylaryl-polyglykoläther auf und gibt 975 Gewichtsteile Wasser hinzu. Das Konzentrat verdünnt man mit Wasser auf die gewünschte Endkonzentration in der Spritzbrühe.

Zur Prüfung auf protektive Wirksamkeit inokuliert man einblättrige Weizenjungpflanzen der Sorte Michigan Amber mit einer Uredosporensuspension von Puccinia recondita in 0,1%igem Wasseragar. Nach Antrocknen der Sporensuspension besprüht man die Weizenpflanzen mit der Wirkstoffzubereitung taufeucht und stellt sie zur Inkubation für 24 Stunden bei etwa 20°C und einer 100%igen Luftfeuchtigkeit in ein Gewächshaus.

Zur Prüfung auf kurative Wirksamkeit geht man in entsprechender Weise vor, mit dem einen Unterschied, daß die Behandlung der Weizenpflanzen mit der Wirkstoffzubereitung erst 48 Stunden nach der Inokulation erfolgt, wenn die Infektion bereit manifest ist.

Nach 10 Tagen Verweilzeit der Pflanzen bei einer Temperatur von 20°C und einer Luftfeuchtigkeit von 80 bis 90% wertet man den Besatz der Pflanzen mit Rostpusteln aus.

Der Befall wird in Prozenten des Befalls der unbehandelten Kontrollpflanzen ausgedrückt. Dabei bedeutet 0% keinen Befall und 100% den gleichen Befallsgrad wie bei der unbehandelten Kontrolle. Der Wirkstoff ist umso wirksamer, je geringer der Rostbefall ist.

Die Versuchsauswertung ergab, daß z.B. die folgenden Verbindungen eine im Vergleich zum Stande der Technik überlegene Wirkung aufweisen.

Verbindungen gemäß Herstellungsbeispielen 2, 3, 1, 9, 10, 15, 16, 17, 18, 19, 20, 33, 22, 34, 25, 26, 35.

## Beispiel F

Sproßbehandlungs-Test/Getreidemehltau/protektiv (blattzerstörende Mykose)

Zur Herstellung einer zweckmäßigen Wirkstoffzubereitung nimmt man 0,25 Gewichtsteile Wirkstoff in 25 Gew.-Teilen Dimethylformamid und 0,06 Gewichtsteilen Emulgator (Alkyl-aryl-polyglykolähter) auf und gibt 975 Gewichtsteile Wasser hinzu. Das Konzentrat verdünnt mit Wasser auf die gewünschte Endkonzentration der Spritzbrühe.

Zur Prüfung auf protektive Wirksamkeit besprüht man die einblättrige Gerstenjungpflanzen der Sorte Amsel mit der Wirkstoffzubereitung taufeucht. Nach Antrocknen besträubt man die Gerstenpflanzen mit Sporen von Erysiphe graminis var. hordei.

Zur Prüfung auf kurative Wirksamkeit geht man in entsprechender Weise aber umgekehrter Reihenfolge vor. Die Behandlung der einblättigen Gerstenjungpflanzen mit der Wirkstoffzubereitung erfolgt 48 Stunden nach der Inokulation, wenn die Infektion bereits manifest ist.

Nach 6 Tagen Verweilzeit der Pflanzen die einer Temperatur von 21 bis 22°C und einer Luftfeuchtigkeit von 80 bis 90% wertet man den Besatz der Pflanzen mit Mehltaupusteln aus. Der Befallgrad wird in Prozenten des Befalls der unbehandelten Kontrollpflanzen ausgedrückt. Dabei bedeutet 0% keinen Befall und 100% den gleichen Befall wie bei der unbehandelten Kontrolle. Der Wirkstoff ist umso wirksamer, je geringer der Mehltaubefall ist.

Die Versuchsauswertung ergab, daß z.B. die folgenden Verbindungen eine im Vergleich zu den beim Stand der Technik genannten Verbindungen überlegene Wirkung aufweisen:

Verbindungen gemäß Herstellungsbeispiel 34.

## Beispiel G

Saatgutbeizmittel-Test/Weizensteinbrand (samenbürtige Mykose)

Zur Herstellung eines zweckmäßigen Trockenbeizmittels verstreckt man den Wirkstoff mit einem Gemisch aus gleichen Gewichtsteilen Talkum und Kieselgur zu einer feinpulverigen Mischung mit der gewünschten Wirkstoffkonzentration.

Man kontaminiert Weizensaatgut mit 5 g Chlamydosporen von Tilletia caries pro kg Saatgut. Zur Beizung schüttelt man das Saatgut mit dem Beizmittel in einer verschlossenen Glasflasche. Das Saatgut wird auf feuchtem Lehm unter einer Deckschichte aus einer Lage Müll und 2 cm mäßig feuchter Komposterde 10 Tage lang im Kühlschrank bei 10°C optimalen Keimungsbedingungen für die Sporen ausgesetzt.

Anschließend bestimmt man mikroskopisch die Keimung der Sporen auf den Weizenkörnern, die jeweils mit rund 100 000 Sporen besetzt sind. Der Wirkstoff ist umso wirksamer je weniger Sporen gekeimt sind.

Die Versuchsauswertung ergab, daß z.B. die folgenden Verbindungen eine im Vergleich zu den beim Stande der Technik genannten Verbindungen überlegene Wirkung aufweisen:

Verbindungen gemäß Herstellungsbeispielen 1, 17, 22.

## Herstellungsbeispiele
### Beispiel 1

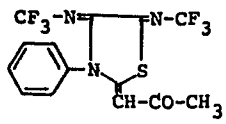

Zu einer Suspension von 19,3 g (0,1 Mol) Acetessigsäurethioanilid und 15 g (0,36 Mol) Natriumfluorid in 120 ml Aceton werden unter Eiskühlung und Rühren bei etwa 5 bis 15°C 23 g (ca. 0,1 Mol) Bis-trifluormethyliminooxalsäuredifluorid zugetropft. Man rührt 2 Stunden bei Raumtemperatur, saugt ab und wäscht mit warmen Acetonitril nach. Dann gießt man auf Eis, saugt ab, wäscht mit reichlich Wasser nach, und kristallisiert den Rückstand aus Äthylalkohol um. Man erhält 32 g 2-Acetylmethylen-3-phenyl-4,5-bis-trifluormethylimino-thiazolidin vom Fp. 189—191°C, das sind 84% der Theorie.

Darstellung des Vorproduktes:

160 ml Acetylaceton und 400 ml absoluter Äther und 23 g fein geschnittenes Natrium werden bei Raumtemperatur während 2 1/2 Stunden gerührt. Sodann tropft man bei Raumtemperatur 135 g Phenylsenföl hinzu und rührt während 60 Stunden bei Raumtemperatur nach. Anschließend tropft man 120 ml absolutes Methanol hinzu und läßt 20 Stunden bei Raumtemperatur stehen. Man nimmt das Gemisch in 200 ml Eiswasser, dem 200 ml 10%ige Natronlauge beigegeben sind, auf, trennt den Äther ab, und neutralisiert die wäßrige Lösung unter Kühlung bei ca. 0°C mit 10%iger Salzäure. Der Niederschlag wird abgesaugt, mit Wasser gewaschen, getrocknet und aus Tetrachlorkohlenstoff umkristallisiert. Man erhält 157 g Acetessigsäure-thioanilid vom Fp. 56—58°C.

In entsprechender Weise, wie in Beispiel 1 angebenen, werden die folgen Verbindungen der allgemeinen Formel

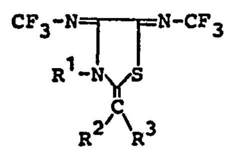 (I)

erhalten:

| Beispiel Nr. | $R^1$ | $R^2$ | $R^3$ | Fp (°C) |
|---|---|---|---|---|
| 2 | 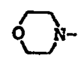 | H | CO—$OCH_3$ | 165—167 |
| 3 | 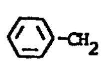 | H | CO—$OCH_3$ | 164—165 |
| 4 | 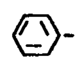 | CO—$OC_2H_5$ | CO—$OC_2H_5$ | 220—222 |
| 5 | 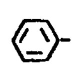 | H | CO—NH-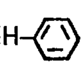 | 224—225 |
| 6 | 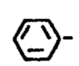 | CN | $SO_2$—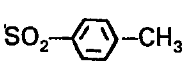 | 286—287 |
| 7 | 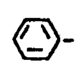 | CN | CO—$OC_2H_5$ | 205—206 |
| 8 | 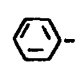 | H | CS—NH-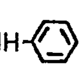 | 207 |
| 9 | 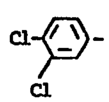 | H | CO—$OCH_3$ | 151—152 |
| 10 | 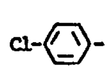 | H | CO—$OCH_3$ | 167—168 |
| 11 | 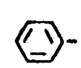 | H | CO—NH-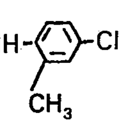 | 226—227 |

| Beispiel Nr. | $R^1$ | $R^2$ | $R^3$ | Fp (°C) |
|---|---|---|---|---|
| 12 | 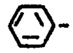 | H | CO—NH 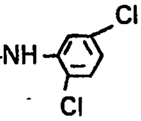 | 196—197 |
| 13 | 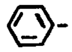 | H | CO—NH 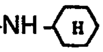 | 224—225 |
| 14 | 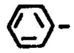 | H | CO—NH—$CH_2$ 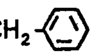 | 237—238 |
| 15 | 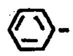 | H | CO—$OCH_3$ | 176—177 |
| 16 | 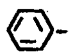 | H | CO—$OC_2H_5$ | 214—215 |
| 17 | 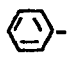 | H | CN | 218—219 |
| 18 | 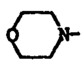 | H | CO—$OC_2H_5$ | 193—194 |
| 19 | 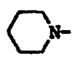 | H | CO—$OC_2H_5$ | 167—168 |
| 20 | 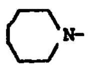 | H | CO—$OCH_3$ | 136—137 |
| 21 | 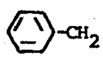 | H | CO—$OC_2H_5$ | 166—167 |
| 22 | 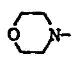 | H | CO—$OC_2H_4OCH_3$ | 128—130 |
| 23 | 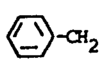 | H | CO—$OC_2H_4OCH_3$ | 146—147 |
| 24 | 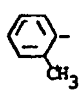 | H | CO—$OCH_3$ | 227—228 |
| 25 | 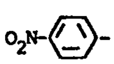 | H | CO—$OCH_3$ | 217—218 |
| 26 | 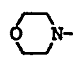 | H | CO—$OC_3H_7$-n | 127—128 |
| 27 | 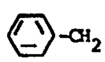 | H | CO—$OC_3H_7$-n | 137—138 |
| 28 | 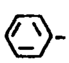 | H | CO 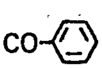 | 203—204 |
| 29 | 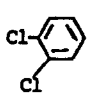 | H | CO 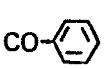 | 187—188 |
| 30 | 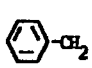 | H | CO 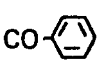 | 200—202 |
| 31 | 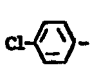 | H | CO—$CH_3$ | 220—221 |
| 32 | 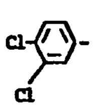 | H | CO—$CH_3$ | 202—203 |

| Beispiel Nr. | $R^1$ | $R^2$ | $R^3$ | Fp (°C) |
|---|---|---|---|---|
| 33 | 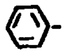 | | $CH_2—CH_2—CH_2—CH_2—CO$ | 157—158 |
| 34 | 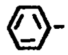 | | $CH_2—CH_2—CH_2—CO$ | 169—170 |
| 35 | 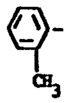 | | $CH_2—CH_2—CH_2—CH_2—CO$ | 162—163 |

**Patentansprüche**

1. Trifluormethylimino-thiazolidin-Derivate der allgemeinen Formel

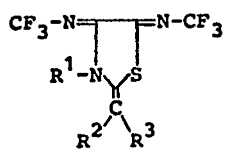

(I)

in welcher

$R^1$ für Alkyl, Alkenyl und Alkinyl steht, wobei diese Gruppen gegebenenfalls substituiert sein können durch Cyano-, Alkoxy- und Alkylmercapto-Gruppen mit jeweils bis zu 3 Kohlenstoffatomen, ferner für gegebenenfalls durch Alkyl mit bis zu 3 Kohlenstoffatomen substituiertes Cycloalkyl steht, ferner für Aryl und Aralkyl steht, wobei die beiden letztgenannten Gruppen am Aryl-Rest substituiert sein können durch Halogen-, Cyano-, Nitro-, Alkyl-, Alkoxy- und Alkylmercapto-Gruppen mit jeweils bis zu 3 Kohlenstoffatomen, und endlich für eine Amino-Gruppe $—NR^4R^5$ steht, wobei

$R^4$ und $R^5$ für Alkyl stehen, wobei die Reste $R^4$ und $R^5$ auch gemeinsam mit dem verbindenden Stickstoffatom und gegebenenfalls noch weiteren Heteroatomen, wie Sauerstoff, Schwefel oder Stickstoff, einen 4- bis 12-gliedrigen heterocyclischen Ring bilden können,

$R^2$ für Wasserstoff, eine Nitril-, Alkoxycarbonyl- oder Acyl-Gruppe steht, und

$R^3$ für eine Nitril- oder Alkoxycarbonyl-Gruppe steht, wobei letztere im Alkylteil substituiert sein kann durch Alkoxy- oder Alkylmercapto-Gruppen mit jeweils bis zu 3 Kohlenstoffatomen, ferner für eine Acyl-, Alkylsulfonyl- oder Arylsulfonyl-Gruppe steht, wobei letztere im Aryl-Teil substituiert sein kann durch Alkyl mit bis zu 3 Kohlenstoffatomen, Nitro-Gruppen und/oder Halogen, ferner für eine Amid- oder Thioamid-gruppe mit den Resten $CO—NR^6R^7$ bzw. $CS—NR^6R^7$ steht, wobei

$R^6$ für Wasserstoff oder Alkyl steht, und

$R^7$ für Alkyl, Cycloalkyl, Benzyl oder Phenyl steht, wobei letzteres substituiert sein kann durch Halogen, Nitro-, Alkyl-, Alkoxy- und Alkylmercapto-Gruppen mit jeweils bis zu 4 Kohlenstoffatomen im Alkylteil, und weiterhin können

$R^2$ und $R^3$ noch gemeinsam mit dem verbindenden Kohlenstoffatom für ein $\alpha$-Cycloalkanon-Ringsystem mit 5 bis 7 Kohlenstoffatomen stehen das gegebenenfalls durch Alkyl mit bis zu 3 Kohlenstoffatomen substituiert sein kann.

2. Verfahren zur Herstellung von Trifluoromethyliminothiazolidin-Derivaten, gemäß Formel I in Anspruch 1, dadurch gekennzeichnet, daß man Thioamide

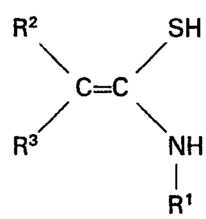

(II)

in welcher

$R^1$ $R^2$ und $R^3$ die in Anspruch 1 angegebene Bedeutung besitzen, mit Bis-trifluormethylimino-oxalsäuredifluorid der Formel

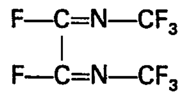

(III)

in Gegenwart eines Fluorwasserstoff-Akzeptors umsetzt.

3. Fungizide Mittel, gekennzeichnet durch einen Gehalt an mindestens einem Trifluormethylimino-thiazolidin-Derivat gemäß Anspruch 1.

4. Verwendung von Trifluormethylimino-thiazolidin-Derivaten gemäß Anspruch 1 zur Bekämpfung von Pilzen.

5. Verfahren zur Herstellung von fungiziden Mitteln, dadurch gekennzeichnet, daß man Trifluormethylimino-thiazolidin-Derivate gemäß Anspruch 1 mit Streckmitteln und/oder oberflächenaktiven Mitteln vermischt.

**Claims**

1. Trifluoromethylimino-thiazolidine derivatives of the general formula

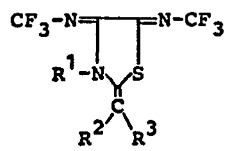

(I)

in which

$R^1$ represents alkyl, alkenyl and alkinyl, it being possible for these groups to be optionally substituted by cyano, alkoxy and alkylmercapto groups with in each case up to 3 carbon atoms, and furthermore represents cycloalkyl optionally substituted by alkyl with up to 3 carbon atoms, and also represents aryl and aralkyl, it being possible for the two last-mentioned groups to be substituted on the aryl radical by halogen, cyano, nitro, alkyl, alkoxy and alkylmercapto groups with in each case up to 3 carbon atoms, and finally represents an amino group $—NR^4R^5$, wherein

$R^4$ and $R^5$ represent alkyl, it being possible for the radicals $R^4$ and $R^5$, together with the connecting nitrogen atom and optionally still further hetero-atoms, such as oxygen, sulphur or nitrogen, to form a 4-membered to 12-membered heterocyclic ring,

$R^2$ represents hydrogen or a nitrile, alkoxycarbonyl or acyl group and

$R^3$ represents a nitrile or alkoxycarbonyl group, it being possible for the latter to be substituted in the alkyl part by alkoxy or alkylmercapto groups with in each case up to 3 carbon atoms, and furthermore represents an acyl, alkylsulphonyl or arylsulphonyl group, it being possible for the latter to be substituted in the aryl part by alkyl with up to 3 carbon atoms, nitro groups and/or halogen, and furthermore represents an amide or thioamide group, comprising the radicals $CO—NR^6H^7$ or $CS—NR^6R^7$, wherein

$R^6$ represents hydrogen or alkyl and

$R^7$ represents alkyl, cycloalkyl, benzyl, or phenyl, it being possible for the latter to be substituted by halogen or nitro, alkyl, alkoxy and alkylmercapto groups with in each case up to 4 carbon atoms in the alkyl part, and furthermore

$R^2$ and $R^3$, together with the connecting carbon atom, can also represent an $\alpha$-cycloalkanone ring system with 5 to 7 carbon atoms, which can be optionally substituted by alkyl with up to 3 carbon atoms.

2. Process for the preparation of trifluoromethylimino-thiazolidine derivatives, according to the formula I in claim 1, characterised in that thioamides

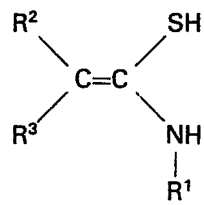

(II)

in which

$R^1$, $R^2$ and $R^3$ have the meaning indicated in claim 1, are reacted with bis-trifluoromethylimino-oxalic acid difluoride of the formula

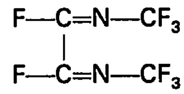

(III)

in the presence of a hydrogen fluoride-acceptor.

3. Fungicidal agents, characterised in that they contain at least one trifluoromethylimino-thiazolidine derivative according to Claim 1.

4. Use of trifluoromethylimino-thiazolidine derivatives according to Claim 1 for combating fungi.

5. Process for the preparation of fungicidal agents, characterised in that trifluoromethylimino-thiazolidine derivatives according to Claim 1 are mixed with extenders and/or surface-active agents.

**Revendications**

1. Dérivés de la trifluorométhylimino-thiazolidine, répondant à la formule générale

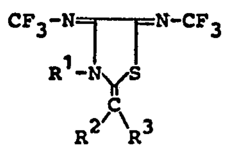

(I)

dans laquelle

$R^1$ représente un groupe alkyle, alcényle ou alcynyle, ces groupes pouvant le cas échéant être substitués par des groupes cyano, alcoxy et alkylmercapto contenant chacun jusqu'à 3 atomes de carbone, un groupe cycloalkyle éventuellement substitué par un groupe alkyle contenant jusqu'à 3 atomes de carbone, un groupe aryle ou aralkyle, ces deux derniers groupes pouvant être substitués sur le radical aryle par des halogènes, des groupes cyano, nitro, alkyle, alcoxy et alkylmercapto contenant chacun jusqu'à 3 atomes de carbone, et finalement un groupe amino-$NR^4R^5$ dans lequel,

$R^4$ et $R^5$ représentent des groupes alkyle ou forment en commun et avec l'atome d'azote qui les relie et le cas échéant d'autres hétéroatomes comme l'oxygène, le soufre ou l'azote, un noyau hétérocyclique de 4 à 12 chaînons,

$R^2$ représente l'hydrogène, un groupe nitrile, alcoxycarbonyle ou acyle, et

$R^3$ représente un groupe nitrile ou alcoxycarbonyle, ce dernier pouvant être substitué dans la partie alkyle par des groupes alcoxy ou alkylmercapto contenant chacun jusqu'à 3 atomes de carbone, un groupe acyle, alkylsulfonyle ou arylsulfonyle, ce dernier pouvant être substitué dans la partie aryle par un groupe alkyle contenant jusqu'à 3 atomes de carbone, des groupes nitro et/ou un halogène, un groupe amide ou thioamide avec les restes $CO—NR^6R^7$ ou respectivement $CS—NR^6R^7$ dans lesquels

$R^6$ représente l'hydrogène ou un groupe alkyle, et

$R^7$ représente un groupe alkyle, cycloalkyle, benzyle ou phényle, ce dernier pouvant être substitué par un halogène des groupes nitro, alkyle, alcoxy et alkylmercapto contenant chacun jusqu'à 4 atomes de carbone dans la partie alkyle, et en outre

$R^2$ et $R^3$ peuvent encore former en commun et avec l'atome de carbone qui les relie un système cyclique $\alpha$-cycloalcanone contenant de 5 à 7 atomes de carbone et qui peut le cas échéant être substitué par un groupe alkyle contenant jusqu'à 3 atomes de carbone.

2. Procédé de préparation des dérivés de la trifluorométhylimino-thiazolidine de formule I selon la revendication 1, caractérisé en ce que l'on fait réagir des thioamides

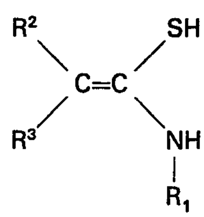

(II)

dans lesquels

$R^1$, $R^2$ et $R^3$ ont les significations indiquées dans la revendication 1, avec le difluorure de l'acide bis-trifluorométhylimino-oxalique de formule

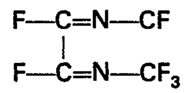

(III)

en présence d'un accepteur de fluorure d'hydrogène.

3. Produit fongicide, caractérisé en ce qu'il contient au moins un dérivé de la trifluorométhyliminothiazolidine selon la revendication 1.

4. Utilisation des dérivés de la trifluorométhyliminothiazolidine selon la revendication 1 pour la lutte contre les mycètes.

5. Procédé de préparation de produits fongicides, caractérisé en ce que l'on mélange des dérivés de la trifluorométhyliminothiazolidine selon la revendication 1 avec des diluants et/ou des agents tensioactifs.